(19) Europäisches Patentamt
European Patent Office
Office européen des brevets

(11) EP 1 533 301 B1

(12) EUROPÄISCHE PATENTSCHRIFT

(45) Veröffentlichungstag und Bekanntmachung des Hinweises auf die Patenterteilung:
**26.08.2009 Patentblatt 2009/35**

(51) Int Cl.:
***C07D 229/00** (2006.01)* ***C08G 18/02** (2006.01)*

(21) Anmeldenummer: **04026390.7**

(22) Anmeldetag: **06.11.2004**

(54) **Herstellung uretdiongruppenhaltiger Polyisocyanate mit Bicycloalkylphosphinen als Katalysatoren**

Preparation of polyisocyanates containing uretdione groups with bicycloalkylphosphines as catalysts

Préparation de polyisocyanates contenant des groupes uretdione en présence d'un catalyseur bicycloalkylphosphine

(84) Benannte Vertragsstaaten:
**AT BE BG CH CY CZ DE DK EE ES FI FR GB GR HU IE IS IT LI LU MC NL PL PT RO SE SI SK TR**

(30) Priorität: **21.11.2003 DE 10354544**

(43) Veröffentlichungstag der Anmeldung:
**25.05.2005 Patentblatt 2005/21**

(73) Patentinhaber: **Bayer MaterialScience AG**
**51368 Leverkusen (DE)**

(72) Erfinder:
- **Richter, Frank, Dr.**
**51373 Leverkusen (DE)**
- **Halpaap, Reinhard, Dr.**
**51519 Odenthal (DE)**
- **Laas, Hans-Josef, Dr.**
**51467 Bergisch Gladbach (DE)**
- **Hecking, Andreas**
**40764 Langenfeld (DE)**

(56) Entgegenhaltungen:
**EP-A- 0 780 377** **EP-A- 0 789 017**
**EP-A- 1 174 428** **EP-A- 1 422 223**

- **LAAS H J ET AL: "Zur Synthese aliphatischer Polyisocyanate-Lackpolyisocyanate mit Biuret-, Isocyanurat- oder Uretdionstruktur" JOURNAL FUR PRAKTISCHE CHEMIE, CHEMIKER ZEITUNG, WILEY VCH, WEINHEIM, DE, Bd. 336, Nr. 3, Januar 1994 (1994-01), Seiten 185-200, XP000441642 ISSN: 1436-9966**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die Verwendung von speziellen Cycloalkylphosphinen als Katalysatoren für die Isocyanatdimerisierung (Uretdionbildung) und ein Verfahren zur Herstellung hoch uretdiongruppenhaltiger Polyisocyanate.

Uretdiongruppen aufweisende aliphatische Isocyanate auf Basis gegebenenfalls verzweigter, linear-aliphatischer Diisocyanate zeichnen sich durch eine besonders niedrige Viskosität aus. Produkte auf Basis cycloaliphatischer Diisocyanate sind in der Regel hochviskose bis feste Substanzen, die als abspalterfreie, intern blockierte Vernetzer in Beschichtungssystemen eingesetzt werden können.

Eine Übersicht zur Isocyanat-Oligomerisierung wird in J. Prakt. Chem./Chem. Ztg. 1994, 336, 185-200 gegeben.

Tris(dialkylamino)phosphine (DE-A 3 030 513) gegebenenfalls in Verbindung mit Cokatalysatoren (DE-A 3 437 635) weisen eine gute Selektivität für die Bildung von Uretdiongruppen (Uretdionselektivität) auf. Ihrer technischen Einsetzbarkeit steht allerdings der schwerwiegende Makel des hohen krebserzeugenden Potenzials ihrer Phosphoroxide, z.B. Hexamethylphosphorsäuretriamid, entgegen.

DE-A 3 739 549 offenbart die katalytische NCO-Dimerisierung mit 4-Dialkylaminopyridinen, wie z.B. 4-Dimethylaminopyridin (DMAP), wobei allerdings nur im Fall spezieller cycloaliphatischer Isocyanate wie Isophorondiisocyanat (IPDI) die Uretdionbildung selektiv verläuft. Linearaliphatische Isocyanate wie Hexamethylendiisocyanat (HDI) sowie verzweigte, linearaliphatische Isocyanate wie Trimethylhexandiisocyanat (TMDI) und Methylpentandiisocyanat (MPDI) liefern mit DMAP und verwandten Verbindungen hauptsächlich stark gefärbte, heterogene Reaktionsprodukte.

DE-A 1 670 720 offenbart die Herstellung von Uretdiongruppen aufweisenden aliphatischen Polyisocyanaten, wobei als Katalysatoren tertiäre Phosphine mit mindestens einem aliphatischen Substituenten sowie Bortrifluorid und seine Addukte eingesetzt werden. Es wird darauf hingewiesen, dass nur bei niedrigen Umsätzen und Reaktionstemperaturen zwischen 50 und 80°C hohe Anteile an Uretdiongruppen im Produkt erhalten werden können, wobei gleichzeitig Isocyanat-Trimere (Isocyanurate und Iminooxadiazindione) und, insbesondere bei höherer Temperatur, auch andere Nebenprodukte wie Carbodiimide oder Uretonimine gebildet werden. Uretonimine sind ganz besonders störend, da sie bei Lagerung zur Freisetzung von monomerem Isocyanat neigen.

DE-A-102 54 878 beschreibt die Verwendung von Phosphinen, die mindestens einen cycloaliphatischen, P-gebundenen Rest aufweisen als Katalysatoren für die NCO-Dimerisierung. Diese Katalysatoren zeichnen sich durch eine wesentlich höhere Uretdionselektivität verglichen mit anderen Trialkylphosphinen des Standes der Technik aus.

Es wurde nun gefunden, dass Phosphine, die mindestens einen direkt Phosphor-gebundenen bicyclischen, cycloaliphatischen Rest aufweisen, ebenfalls als Katalysatoren für die selektive Uretdion-Bildung (Isocyanat-Dimerisierung) gut geeignet sind. Darüber hinaus weisen die erfindungswesentlichen Phosphine bei gleicher Anzahl cyclischer P-gebundener Substituenten eine höhere Selektivität und Katalysatorstandzeit auf als ihre Analoga mit monocyclischen Substituenten.

Gegenstand der Erfindung ist die Verwendung von Phosphinen, die mindestens einen, direkt Phosphor-gebundenen bicyclischen, cycloaliphatischen Rest aufweisen, bei der Dimerisierung von Isocyanaten.

Phosphine zur Isocyanat-Dimerisierung entsprechen der Formel I:

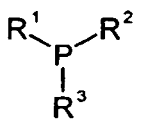

(I)

wobei

$R^1$ ein gegebenenfalls ein oder mehrfach $C_1$-$C_{12}$ Alkyl- oder Alkoxy-substituierter, bicyclischer, cycloaliphatischer $C_4$-$C_{20}$-Rest ist und

$R^2$, $R^3$ unabhängig voneinander ein gegebenenfalls ein oder mehrfach $C_1$-$C_{12}$ Alkyl- oder Alkoxy-substituierter mono- oder bicyclischer, cycloaliphatischer $C_4$-$C_{20}$-Rest oder ein linear oder verzweigt aliphatischer $C_1$-$C_{20}$-Rest ist.

Bevorzugt sind Verbindungen der Formel I bei denen $R^1$ für einen ein- oder mehrfach $C_1$-$C_{12}$ Alkyl-substituierten Norbornylrest (= 2,2,1-Bicycloheptylrest) steht und $R^2$ wahlweise identisch zu $R^1$ oder zu $R^3$ ist und wobei $R^3$ für einen ein- oder mehrfach $C_1$-$C_8$ Alkyl-substituierten, aliphatischen $C_1$-$C_{12}$-Alkylrest steht.

Beispiele erfindungsgemäß einzusetzender Phosphine sind: Norbornyl-dimethylphosphin, Norbornyl-diethylphosphin, Norbornyl-di-n-propylphosphin, Norbornyl-di-isopropylphosphin, Norbomyldibutylphosphin wobei 'Butyl' für alle Isomeren, d.h. n-Butyl, iso-Butyl, 2-Butyl, tert.-Butyl sowie cyclo-Butyl stehen kann, Norbornyl-dihexylphosphin (alle isomeren Hexylreste), Norbomyldioctylphosphin (alle isomeren Octylreste), Dinorbomyl-methylphosphin, Dinorbomyl-ethylphosphin, Dinorbomyl-n-propylphosphin, Dinorbornyl-isopropylphosphin, Dinorbomyl-butylphosphin (alle isomeren Butylreste), Dinorbornyl-hexylphosphin (alle isomeren Hexylreste), Dinorbornyl-octylphosphin (alle isomeren Octylreste), Trinorbornylphosphin, Dimethyl-(1,7,7-trimethyl-bicyclo[2.2.1]hept-2-yl)-phosphin, Diethyl-(1,7,7-trimethyl-bicyclo [2.2.1]hept-2-yl)-phosphin, Di-n-propyl-(1,7,7-trimethyl-bicyclo[2.2.1]hept-2-yl)-phosphin, Di-iso-propyl-(1,7,7-trimethyl-bicyclo[2.2.1]hept-2-yl)-phosphin, Dibutyl-(1,7,7-trimethyl-bicyclo[2.2.1]hept-2-yl)-phosphin (alle isomeren Butylreste), Dihexyl-(1,7,7-trimethyl-bicyclo[2.2.1]hept-2-yl)-phosphin (alle isomeren Hexylreste), Dioctyl-(1,7,7-trimethyl-bicyclo [2.2.1]hept-2-yl)-phosphin (alle isomeren Octylreste), Methyl-bis-(1,7,7-trimethyl-bicyclo[2.2.1]hept-2-yl)-phosphin, Ethyl-bis-(1,7,7-trimethyl-bicyclo[2.2.1]hept-2-yl)-phosphin, n-Propyl-bis-(1,7,7-trimethyl-bicyclo[2.2.1]hept-2-yl)-phosphin, iso-Propyl-bis-(1,7,7-trimethyl-bicyclo[2.2.1]hept-2-yl)-phosphin, Butyl-bis-(1,7,7-trimethyl-bicyclo[2.2.1]hept-2-yl)-phosphin (alle isomeren Butylreste), Hexyl-bis-(1,7,7-trimethylbicyclo[2.2.1]hept-2-yl)-phosphin (alle isomeren Hexylreste), Octyl-bis-(1,7,7-trimethylbicyclo[2.2.1]hept-2-yl)-phosphin (alle isomeren Octylreste), Dimethyl-(2,6,6-trimethyl-bicyclo-[3.1.1]hept-3-yl)-phosphin, Diethyl-(2,6,6-trimethyl-bicyclo[3.1.1]hept-3-yl)-phosphin, Di-npropyl-(2,6,6-trimethyl-bicyclo[3.1.1]hept-3-yl)-phosphin, Di-iso-propyl-(2,6,6-trimethyl-bicyclo-[3.1.1]hept-3-yl)-phosphin, Dibutyl-(2,6,6-trimethyl-bicyclo[3.1.1]hept-3-yl)-phosphin (alle isomeren Butylreste), Dihexyl-(2,6,6-trimethyl-bicyclo[3.1.1] hept-3-yl)-phosphin (alle isomeren Hexylreste), Dioctyl-(2,6,6-trimethyl-bicyclo[3.1.1]hept-3-yl)-phosphin (alle isomeren Octylreste), Methyl-bis-(2,6,6-trimethyl-bicyclo[3.1.1]hept-3-yl)-phosphin, Ethyl-bis-(2,6,6-trimethyl-bicyclo-[3.1.1]hept-3-yl)-phosphin, n-Propyl-bis-(2,6,6-trimethyl-bicyclo[3.1.1]hept-3-yl)-phosphin, iso-Propyl-bis-(2,6,6-trimethyl-bicyclo [3.1.1]hept-3-yl)-phosphin, Butyl-bis-(2,6,6-trimethylbicyclo[3.1.1]hept-3-yl)-phosphin (alle isomeren Butylreste), Hexyl-bis-(2,6,6-trimethylbicyclo[3.1.1]hept-3-yl)-phosphin (alle isomeren Hexylreste), sowie Octyl-bis-(2,6,6-trimethylbicyclo [3.1.1]hept-3-yl)-phosphin (alle isomeren Octylreste).

Diese können als Katalysator für die Uretdionbildung einzeln, in beliebigen Mischungen untereinander oder in Mischungen mit anderen primären, sekundären und/oder tertiären Alkyl-, Aralkylund/oder Arylphosphinen verwendet werden.

Ein weiterer Gegenstand der Erfindung ist ein Verfahren zur Herstellung uretdiongruppenhaltiger Polyisocyanate, bei dem

a) mindestens ein organisches Isocyanat einer NCO-Funktionalität ≥2,
b) ein Katalysator enthaltend mindestens ein erfindungsgemäß einzusetzendes Phosphin,
c) optional Lösemittel und
d) optional Additive

zur Reaktion gebracht werden.

Die Menge des im erfindungsgemäßen Verfahren einzusetzenden Katalysators richtet sich in erster Linie nach der angestrebten Reaktionsgeschwindigkeit und liegt im Bereich 0,01 bis 5 mol-%, bezogen auf die Summe der Stoffmengen des eingesetzten Isocyanates und des Katalysators. Bevorzugt werden 0,05 bis 3 mol-% Katalysator eingesetzt.

Der Katalysator b) kann im erfindungsgemäßen Verfahren unverdünnt oder in Lösungsmitteln gelöst eingesetzt werden. Als Lösungsmittel kommen dabei alle Verbindungen in Frage, die nicht mit Phosphinen reagieren wie z.B. aliphatische oder aromatische Kohlenwasserstoffe, Alkohole, Ketone, Ester sowie Ether. Bevorzugt werden die Phosphine im erfindungsgemäßen Verfahren unverdünnt eingesetzt.

Als in a) erfindungsgemäß einzusetzende Isocyanate können prinzipiell alle bekannten, durch Phosgenierung oder nach phosgenfreien Verfahren hergestellten organischen Isocyanate einzeln oder in beliebigen Mischungen untereinander verwendet werden.

Bevorzugt ist die Verwendung von aliphatischen, cycloaliphatischen oder araliphatischen Di- oder Polyisocyanaten einer NCO-Funktionalität ≥ 2.

Besonders bevorzugt ist die Verwendung gegebenenfalls verzweigter, gegebenenfalls cyclische Reste enthaltender aliphatischer Diisocyanate mit an ein primäres Kohlenstoffatom gebundenen Isocyanatgruppen. Beispiele hierfür sind Pentandiisocyanat, Hexandiisocyanat, Heptandiisocyanat, Octandiisocyanat, Nonandiisocyanat, Dekandiisocyanat, Undekandiisocyanat und Dodecandiisocyanat, wobei beliebige Isomere der vorstehend genannten Verbindungen zum Einsatz kommen können.

Ganz besonders bevorzugt werden Hexamethylendiisocyanat (HDI), Methylpentandiisocyanat (MPDI), Trimethylhexandiisocyanat (TMDI), Bis(isocyanatomethyl)cyclohexan ($H_6$XDI) sowie Norbornandiisocyanat (NBDI) einzeln oder in beliebigen Mischungen untereinander eingesetzt.

Darüber hinaus können Isophorondiisocyanat (IPDI), Bis(isocyanatocyclohexyl)methan ($H_{12}$MDI), Bis(isocyantomethyl)benzol (Xylylendiisocyanat, XDI) und Bis(2-isocyanatoprop-2-yl)benzol (Tetramethylxylylendiisocyanat, TMXDI) im erfindungsgemäßen Verfahren eingesetzt werden.

Das erfindungsgemäße Verfahren wird so geführt, dass der Umsatz der NCO-Gruppen von 5 bis 90 %, bevorzugt von 10 bis 60 %, besonders bevorzugt von 10 bis 50 % beträgt.

Das erfindungsgemäße Verfahren wird im Temperaturbereich 0°C bis 150°C, bevorzugt 0°C bis 80°C, besonders bevorzugt 0°C bis 60°C, insbesondere 0°C bis 40°C durchgeführt.

Um Umsätze der NCO-Gruppen gemäß der vorstehenden Bereiche zu realisieren, wird die Reaktion bei dem gewünschten Umsetzungsgrad abgebrochen.

Zum Abbruch der Reaktion nach Erreichen des gewünschten Umsetzungsgrades eignen sich prinzipiell alle vorbeschriebenen Katalysatorgifte (DE-A 1670667, 1670720, 1934763, 1954093, 3437635, US 4614785) wie Alkylierungsmittel (z.B. Dimethylsulfat, Toluolsulfonsäuremethylester), organische oder anorganische Peroxide, Säurechloride sowie Schwefel, die mit dem Katalysator gegebenenfalls unter Temperaturerhöhung zur Reaktion gebracht werden (Variante A, vgl. auch Beispiele 1 bis 6).

Nach der Desaktivierung der Reaktionsmischung nach Variante A kann nicht umgesetztes Monomer und/oder der deaktivierte Katalysator abgetrennt werden.

Das Verfahren kann auch ohne chemische Deaktivierung des Katalysators durchgeführt werden. Dazu wird unmittelbar nach Erreichen des gewünschten Umsatzes der aktive Katalysator aus der Reaktionsmischung abgetrennt, um eine Weiterreaktion gegebenenfalls unter Nebenproduktbildung zu unterbinden (Variante B).

Gleichzeitig mit oder auch nach der Katalysatorabtrennung kann nicht umgesetztes Restmonomer aus der nach Variante B behandelten Reaktionsmischung abgetrennt werden.

Im erfindungsgemäßen Verfahren können zur Abtrennung nicht umgesetzter Monomere, des Katalysators und/oder anderer unerwünschter Bestandteile aus der Reaktionsmischung alle bekannten Separationstechniken wie z.B. Destillation, Extraktion oder Kristallisation/Filtration verwendet werden. Bevorzugt ist die Destillation, gegebenenfalls in der speziellen Ausführungsform der Dünnschichtdestillation. Selbstverständlich können auch Kombinationen zweier oder mehrerer dieser Techniken angewendet werden.

Bevorzugt wird zum Reaktionsabbruch nach Variante B der Katalysator destillativ entfernt, wobei gleichzeitig gegebenenfalls nicht umgesetztes Monomer mit entfernt wird.

Bevorzugt wird bei der Aufarbeitung einer nach Variante A oder B abgebrochenen Reaktion das enthaltene Restmonomer destillativ entfernt.

Soll das erfindungsgemäß hergestellte Polyisocyanat noch freies, nicht umgesetztes Monomer enthalten, wie es z.B. für die Weiterverarbeitung zu NCO-blockierten Produkten oder NCO-armen bzw. -freien Polyuretdionhärtem z.B. für den Pulverlackbereich von Interesse ist, so kann nach Reaktionsabbruch (Varianten A und B) auf die Monomerenabtrennung verzichtet werden.

Es ist für die Durchführung des erfindungsgemäßen Verfahrens unerheblich, ob das Verfahren ganz oder teilweise diskontinuierlich oder kontinuierlich durchgeführt wird.

Weiterhin können im erfindungsgemäßen Verfahren zu einem beliebigen Zeitpunkt, in der Polyisocyanatchemie übliche Additive und Stabilisatoren zugesetzt werden. Beispiele sind Antioxidanzien, wie z.B. sterisch gehinderte Phenole (2,6-Di-tert-butylphenol, 4-Methyl-2,6-di-tert-butylphenol), Lichtschutzmittel, wie z.B. HALS-Amine, Triazole etc., schwache Säuren oder Katalysatoren für die NCO-OH-Reaktion wie z.B. Dibutylzinndilaurat (DBTL).

Des weiteren kann es sinnvoll sein, einem nach Variante B aufgearbeiteten Produkt, geringe Mengen eines in Variante A zu verwendenden Katalysatorgiftes zuzusetzen, um die Rückspaltstabilität zu erhöhen und die Neigung zur Nebenproduktbildung, zur Verfärbung bzw. zur Weiterreaktion der freien NCO-Gruppen untereinander, z.B. bei Produktlagerung, zu unterdrücken.

Nach dem erfindungsgemäßen Verfahren hergestellte Produkte auf Basis gegebenenfalls verzweigter, linearaliphatischer Di- oder Polyisocyanate, die keine Cycloalkylsubstituenten aufweisen, sind farbhell und haben eine Viskosität < 1000 mPas/23°C. Werden cycloaliphatische und/oder araliphatische Di- oder Polyisocyanate eingesetzt, werden hochviskose bis feste Harze erhalten (Viskosität > 10000 mPas/23°C).

In monomerenarmer Form, d.h. nach Abtrennung von nicht umgesetztem Monomer, weisen die erfindungsgemäß hergestellten Produkte einen NCO-Gehalt < 27,3 Gew.-%, bevorzugt < 25 Gew.-%, auf.

Die nach dem erfmdungsgemäßen Verfahren hergestellten Polyisocyanate dienen als Ausgangsmaterialien zur Herstellung von z.B. Formkörpern (gegebenenfalls geschäumt), Lacken, Beschichtungsmitteln, Klebstoffen, Dichtmassen oder Zuschlagstoffen, wobei die enthaltenen freien, nicht uretdionisierten NCO-Gruppen auch gegebenenfalls blockiert sein können.

Zur Blockierung der freien, nicht uretdionisierten NCO-Gruppen eignen sich alle dem Fachmann bekannten Methoden. Als Blockierungsmittel können insbesondere Phenole (z.B. Phenol, Nonylphenol, Kresol), Oxime (z.B. Butanonoxim, Cyclohexanonoxim), Lactame (z.B. ε-Caprolactam), sekundäre Amine (z.B. Diisopropylamin), Pyrazole (z.B. Dimethylpyrazol), Imidazole, Triazole oder Malon- und Essigsäureester verwendet werden.

Die nach dem erfindungsgemäßen Verfahren hergestellten, weitgehend nebenproduktfreien, Uretdiongruppen aufweisenden Polyisocyanate können insbesondere zur Herstellung von Ein- und Zweikomponenten-Polyurethanlacken gegebenenfalls in Mischungen mit anderen Di- oder Polyisocyanaten des Standes der Technik, wie Biuret-, Urethan-, Allophanat-, Isocyanurat-, sowie Iminooxadiazindiongruppen enthaltenden Di- oder Polyisocyanaten eingesetzt werden.

Ebenfalls besonders bevorzugt ist die Verwendung der erfindungsgemäß hergestellten Polyisocyanate auf Basis gegebenenfalls verzweigter, linearaliphatischer Isocyanate als Reaktivverdünner zur Viskositätserniedrigung höherviskoser Polyisocyanat-Harze.

Zur Umsetzung der erfindungsgemäß hergestellten Polyisocyanate zum Polyurethan können alle Verbindungen mit mindestens zwei Isocyanat-reaktiven Funktionalitäten einzeln oder in beliebigen Mischungen untereinander (Isocyanat-reaktives Bindemittel) eingesetzt werden.

Bevorzugt ist die Verwendung eines oder mehrerer, in der Polyurethanchemie an sich bekannter, isocyanatreaktiver Bindemittel wie Polyhydroxyverbindungen oder Polyamine. Als Polyhydroxyverbindungen werden besonders bevorzugt Polyester-, Polyether-, Polyacrylat- und/oder Polycarbonsäure-Polyole, gegebenenfalls auch unter Zusatz niedermolekularer, mehrwertiger Alkohole eingesetzt.

Das Äquivalentverhältnis zwischen nicht uretdionisierter Isocyanatgruppe, die gegebenenfalls auch blockiert sein kann, und isocyanatreaktiver Funktionalität des Isocyanat-reaktiven Bindemittels, wie z.B. OH-, NH- oder COOH, liegt von 0,8 bis 3, vorzugsweise 0,8 bis 2.

Möglich ist der Einsatz eines Überschusses an isocyanatreaktivem Bindemittel, da die Spaltung des Uretdionringes gegebenenfalls bei erhöhter Temperatur und/oder Katalysatorzusatz zur Freisetzung weiterer NCO-Gruppen führt, die mit dem Überschuss an isocyanatreaktiven Funktionalitäten reagieren können. Dadurch erhöht sich die Netzwerkdichte des gebildeten Polymers und dessen Eigenschaften werden vorteilhaft beeinflusst.

Für die Beschleunigung der Vemetzungsreaktion der erfindungsgemäß hergestellten Polyisocyanate mit dem Isocyanat-reaktiven Bindemittel können alle aus der Polyurethanchemie bekannten Katalysatoren verwendet werden. Beispielweise können Metallsalze wie Dibutylzinn-IV-dilaurat, Zinn-II-bis(2-ethylhexanoat), Wismut-III-tris(2-ethylhexanoat), Zink-II-bis(2-ethylhexanoat) oder Zinkchlorid sowie tertiäre Amine wie 1,4-Diazabicyclo(2,2,2)oktan, Triethylamin oder Benzyldimethylamin verwendet werden.

Bei der Formulierung werden das erfindungsgemäß hergestellte, gegebenenfalls blockierte Polyisocyanat, das Isocyanat-reaktive Bindemittel, Katalysator(en) und gegebenenfalls die üblichen Zusätze wie Pigmente, Füllstoffe, Additive, Verlaufshilfsmittel, Entschäumer und/oder Mattierungsmittel miteinander auf einem üblichen Mischaggregat wie z.B. einer Sandmühle, gegebenenfalls unter Verwendung von Lösungsmitteln, vermischt und homogenisiert.

Als Lösungsmittel geeignet sind alle an sich bekannten üblichen Lacklösemittel wie z.B. Ethylund Butylacetat, Ethylen- oder Propylenglykolmono-methyl-, -ethyl- oder -propyletheracetat, 2-Butanon, 4-Methyl-2-pentanon, Cyclohexanon, Toluol, Xylol, Solventnaphtha, N-Methylpyrrolidon etc.

Die Beschichtungsmittel können in Lösung oder aus der Schmelze sowie gegebenenfalls in fester Form (Pulverlacke) nach den üblichen Methoden wie z.B. Streichen, Rollen, Gießen, Spritzen, Tauchen, dem Wirbelsinterverfahren oder durch elektrostatische Sprühverfahren auf dem zu beschichtenden Gegenstand appliziert werden.

Als Substrate eignen sich sämtliche bekannten Werkstoffe, insbesondere Metalle, Holz, Kunststoffe und Keramik.

**Beispiele:**

Alle Prozentangaben sind, soweit nicht anders vermerkt, als Gewichtsprozente zu verstehen.

Die Ermittlung des NCO-Gehaltes der in den Beispielen und Vergleichsbeispielen beschriebenen Harze erfolgte durch Titration gemäß DIN 53 185.

Die dynamischen Viskositäten wurden bei 23°C mit dem Viskosimeter VT 550, Fa. Haake, Karlsruhe, DE bestimmt. Durch Messungen bei unterschiedlichen Schergeschwindigkeiten wurde sichergestellt, dass das Fließverhalten der beschriebenen erfindungsgemäß hergestellten Polyisocyanate wie auch das der Vergleichsprodukte dem idealer Newtonscher Flüssigkeiten entspricht. Die Angabe der Schergeschwindigkeit kann deshalb entfallen.

Die Angaben 'mol-%' bzw. 'molares Verhältnis unterschiedlicher Strukturtypen zueinander' basieren auf NMR-spektroskopischen Messungen. Sie beziehen sich immer, wenn nicht anders angegeben, auf die Summe der durch die Modifizierungsreaktion (Oligomerisierung) aus den vorher freien NCO-Gruppen des zu modifizierenden Isocyanates gebildeten Strukturtypen.

$^{13}$C-NMR-Messungen erfolgten auf den Geräten DPX 400, AVC 400 bzw. DRX 700 der Fa. Bruker, Karlsruhe, DE an ca. 50 %-igen Proben in trockenem $CDCl_3$ bzw. an ca. 80 %-igen Proben in $D_6$-DMSO ($^{13}$C-NMR: 100 bzw 176 MHz, relaxation delay: 4 sec, mind. 2000 scans). Als Referenz für die ppm-Skala wurden geringe Mengen Tetramethylsilan im entsprechenden Lösungsmittel ($\delta$ = 0 ppm) oder das Lösungsmittel selbst ($\delta$ = 77,0 ppm ($CDCl_3$) bzw. $\delta$ = 43,5 ppm ($D_6$-DMSO)) gewählt.

Soweit nicht anders angegeben, wurden die Reaktionen mit frisch entgastem HDI als Edukt durchgeführt. Die Bezeichnung ‚frisch entgast' bedeutet dabei, dass das eingesetzte HDI unmittelbar vor der katalytischen Umsetzung durch mindestens 30-minütiges Rühren im Vakuum (< 1 mbar) von gelösten Gasen befreit und anschließend mit Stickstoff belüftet wurde.

Alle Reaktionen wurden unter einer Atmosphäre von trockenem Stickstoff durchgeführt.

Norbornyl- bzw. Di-norbornylsubstituierte Phosphine wurden nach literaturbekannten Methoden (J. Org. Chem., 1961, 26, 513 8 - 5145) durch radikalische Addition von 1-Olefinen an Norbomylphosphin (Bicyclo[2.2.1]hept-2-yl-phos-

phan; $nbPH_2$) bzw. Di-norbomylphosphin (Bis-bicyclo-[2.2.1]hept-2-yl-phosphan; $nb_2PH$) hergestellt. Es kann selbstverständlich auch von Alkyl- bzw. Dialkylphosphinen und Norbomen (Bicyclo[2.2.1]hept-2-en) ausgegangen werden.

**Herstellung von Norbornyl-diethylphosphin ($nbPEt_2$)**

In einen 1,5 1 Rührautoklaven wurden bei Zimmertemperatur unter Stickstoff 108 g einer 50 %igen Lösung von Norbornylphosphin in Toluol (Cytec Canada Inc., Ontario, CA), 3,2 g Azo-bisisobuttersäurenitril (AIBN) und 25 ml Toluol gegeben. Anschließend wurden 32 g Ethylen zudosiert und unter Rühren auf 80°C erwärmt. Nachdem der Druck von anfangs 30 bar innerhalb von 3 h auf 13 bar gefallen ist, wurde auf Zimmertemperatur abgekühlt und entspannt. Anschließend wurden weitere 1,6 g AIBN, gelöst in 40 ml Toluol, und 32 g Ethylen zugefügt und es wurde unter Rühren 5 h auf 70°C erwärmt. Anschließend wurde destillativ im Hochvakuum aufgearbeitet wobei 52 g (95 % d. Theorie; Sdp: 52 - 54°C bei 0,006 mbar) $nbPEt_2$ erhalten wurden.

Analog wurden Norbornyl-dibutyphosphin ($nbPBu_2$, Sdp.: 95°C bei 0,01 mbar) und Dinorbornylethylphosphin ($nb_2PEt$, Sdp.: 125°C bei 0,1 mbar) erhalten. Bei den höheren Homologen Norbornyl-dihexylphosphin ($nbPHex_2$, Sdp.: 150°C bei 0,01 mbar), Norbornyl-didecylphosphin ($nbPDec_2$, Sdp.: 200°C (Badtemperatur bei Kugelrohrdestillation) bei 0,003 mbar) und Dinorbornyldecylphosphin ($nb_2PDec$, Sdp.: 190°C (Badtemperatur bei Kugelrohrdestillation) bei 0,03 mbar) konnte drucklos gearbeitet werden, da die Siedepunkte der entsprechenden Olefine (1-Hexen bzw. 1-Decen) bei Normaldruck hoch genug liegen, um eine ausreichend zügige Zersetzung des AIBN zum Start der Radikalkettenreaktion zu bewirken.

**Beispiele 1 bis 6, erfindungegemäß**

Jeweils 10 g frisch entgastes HDI wurden in mit Septen verschlossenen Glasgefäßen unter Stickstoff in Gegenwart der in Tabellen 1 bis 6 angegebenen Mengen des dort angegebenen Katalysators bei den angegebenen Temperaturen mit einem Magnetrührkern gerührt, wobei in regelmäßigen Abständen der Fortgang der Reaktion durch Messung des Brechungsindex' (bei 20°C und der Frequenz des Lichtes der D-Linie des Natriumspektrums, $n_D^{20}$) der Reaktionsmischung überprüft wurde (Start = kein Umsatz = $n_D^{20}$ des reinen HDI = 1,4523).

Zwischen den Größen Umsatz (Ausbeute) und $n_D^{20}$ der Reaktionsmischung besteht im Ausbeutebereich bis ca. 60 % Uretdion-Polyisocyanatharz in der Reaktionsmischung ein nahezu linearer Zusammenhang (vgl. Beispiel 7 und Figur 2). Die in Beispiel 7 dargestellte Abhängigkeit des Umsatzes vom $n_D^{20}$ der Reaktionsmischung wurde bei den hier diskutierten Proben zur Berechnung des Umsatzes herangezogen; dazu wurde der gemessene Brechungsindex in die Formel

$$\text{Umsatz [\%]} = 19{,}849 * n_D^{20} - 28{,}742$$

eingesetzt und der Umsatz berechnet.

Vor der Selektivitäts-Bestimmung wurden die Umsatzproben zur Unterbindung der Weiterreaktion mit der ihrem Phosphingehalt entsprechenden Menge elementaren Schwefels versetzt und NMRspektroskopisch untersucht. Zur besseren Übersichtlichkeit der Selektivitäten wurde die Größe U/T als das molare Verhältnis der Uretdionstrukturen zur Summe der beiden Trimerstrukturen (Isocyanurat und Iminooxadiazindion) definiert.

**Tabelle 1**: Katalysator: $nbPHex_2$ (0,3 mol-%, bezogen auf HDI) Reaktionstemperatur: 40°C

| Reaktionszeit [hh:mm] | $n_D^{20}$ | HDI-Umsatz [%] | U/T |
|---|---|---|---|
| 01:02 | 1,4528 | 9% | 14,2 |
| 03:28 | 1,4539 | 12% | 9,9 |
| 07:38 | 1,4554 | 15% | 8,8 |
| 23:08 | 1,4589 | 22% | 7,6 |
| 32:00 | 1,4599 | 24% | 7,7 |

**Tabelle 2**: Katalysator: $nbPHex_2$ (0,3 mol-%, bezogen auf HDI) Reaktionstemperatur: 60°C

| Reaktionszeit [hh:mm] | $n_D^{20}$ | HDI-Umsatz [%] | U/T |
|---|---|---|---|
| 01:45 | 1,4536 | 11% | 14,6 |
| 03:45 | 1,4548 | 13% | 13,1 |
| 05:45 | 1,4562 | 16% | 12,1 |
| 21:10 | 1,4632 | 30% | 7,8 |
| 29:25 | 1,4655 | 35% | 6,8 |
| 45:40 | 1,4688 | 41% | 5,5 |
| 69:35 | 1,4706 | 45% | 5,0 |

**Tabelle 3**: Katalysator: $nbPHex_2$ (0,3 mol-%, bezogen auf HDI) Reaktionstemperatur: 80°C

| Reaktionszeit [hh:mm] | $n_D^{20}$ | HDI-Umsatz [%] | U/T |
|---|---|---|---|
| 01:49 | 1,4542 | 12% | 19,3 |
| 03:49 | 1,4559 | 16% | 15,2 |
| 05:48 | 1,4579 | 20% | 12,6 |
| 21:14 | 1,4648 | 33% | 5,9 |
| 29:27 | 1,4665 | 37% | 4,9 |
| 45:29 | 1,4684 | 40% | 4,1 |

**Tabelle 4**:

Katalysator: $nbPBu_2$ (0,6 mol-%, bezogen auf HDI)

Reaktionstemperatur: 40°C

| Reaktionszeit [hh:mm] | $n_D^{20}$ | HDI-Umsatz [%] | U/T |
|---|---|---|---|
| 00:59 | 1,4533 | 10% | 14,7 |
| 02:08 | 1,4542 | 12% | 11,2 |
| 04:56 | 1,4559 | 16% | 10,7 |
| 21:41 | 1,4634 | 31% | 7,4 |
| 29:56 | 1,4656 | 35% | 6,2 |
| 72:11 | 1,4697 | 43% | 4,8 |

**Tabelle 5**: Katalysator: $nbPDec_2$ (0,6 mol-%, bezogen auf HDI) Reaktionstemperatur: 40°C

| Reaktionszeit [hh:mm] | $n_D^{20}$ | HDI-Umsatz [%] | U/T |
|---|---|---|---|
| 00:58 | 1,4533 | 10% | 11,0 |
| 03:53 | 1,4550 | 14% | 9,5 |
| 07:47 | 1,4569 | 18% | 8,5 |
| 23:03 | 1,4639 | 31% | 6,3 |
| 30:43 | 1,4668 | 37% | 5,8 |
| 47:13 | 1,4722 | 48% | 4,7 |
| 71:23 | 1,4776 | 59% | 3,9 |

**Tabelle 6**: Katalysator: $nb_2PDec$ (0,9/1,4 mol-%, bezogen auf HDI) Reaktionstemperatur: 40°C

| Reaktionszeit [hh:mm] | $n_D^{20}$ | HDI-Umsatz [%] | U/T |
|---|---|---|---|
| 01:06* | 1,4537 | 11% | 27,9 |
| 06:16 | 1,4557 | 15% | 20,6 |
| 21:31 | 1,4591 | 22% | 17,9 |
| 46:16 | 1,4636 | 31% | 11,6 |
| 69:46 | 1,4660 | 36% | 9,4 |
| 93:46 | 1,4674 | 38% | 8,7 |

* bis hier 0,9 mol-% Katalysator, anschließend weitere 0,5 mol-% zugegeben

**Beispiel 7, erfindungsgemäß**

Katalysator: $nbPEt_2$ (1 mol-%, bezogen auf HDI); Reaktionstemperatur: 30°C

In einem doppelwandigen, durch einen externen Kreislauf auf 30°C temperierten Planschliffgefäß mit Rührer, an eine Inertgasanlage (Stickstoff/Vakuum) angeschlossenen Rückflusskühler und Thermometer wurden 1050 g HDI vorgelegt und entgast. Nach Belüften mit Stickstoff wurden 11,6 g $nbPEt_2$ zudosiert und die in Tabelle 7 angegebene Zeit bei 30°C gerührt. Der Brechungsindex der Mischung ($n_D^{20}$) stieg dabei auf 1,4671. Anschließend wurde das Reaktionsgemisch ohne vorherige Desaktivierung des Phosphins aufgearbeitet. Die Aufarbeitung erfolgte durch Vakuumdestillation in einem Dünnschichtverdampfer, Typ Kurzwegverdampfer (KWV), mit vorgeschaltetem Vorverdampfer (VV) (Destillationsdaten: Druck: 0,08 mbar, VV-Temperatur: 120°C, HV-Temp.: 150°C, Destillationsdauer: 1 h), wobei nicht umgesetztes Monomer gemeinsam mit dem aktiven Katalysator als Destillat und das Uretdiongruppen enthaltende Polyisocyanatharz als Sumpfprodukt separiert wurden (Startdurchlauf: Beispiel 7-0).

Das den aktiven Katalysator enthaltende Destillat wurde in einer zweite Planschliff-Rührapparatur, die identisch zur ersten aufgebaut war, gesammelt und unmittelbar nach Destillationsende mit frisch entgastem HDI auf die Ausgangsmenge (1050 g) wieder aufgefüllt. Anschließend wurde erneut die in Tabelle 7 angegebene Zeit bei 30°C gerührt und nach Messung des Brechungsindex' der Reaktionsmischung wie oben beschrieben destillativ aufgearbeitet (Beispiel 7-A).

Diese Verfahrensweise wurde insgesamt 18 mal wiederholt (bis Versuch 7-R).

Man beobachtete im Verlauf des über mehrere Wochen durchgeführten Versuches eine nur sehr langsame Abnahme der katalytischen Aktivität (vgl. hierzu Beispiel 8, Vergleichsbeispiel) als deren Maß der Anstieg der $n_D^{20}$/Zeit-Kurve jedes individuellen Versuches herangezogen wurde (Anstieg = ($n_D^{20}$ bei Destillationsbeginn -1,4523)/Reaktionszeit). Dabei wurden die für jeden Versuch erhaltenen Werte zu dem im Startansatz gemessenen (definiert als 100 %) ins Verhältnis gesetzt (vgl. relative Reaktivität in Tabelle 7 und Figur 1).

Durch Korrelation des Brechungsindex' der Rohwaren mit den realisierten Harzausbeuten (= HDI-Umsatz) ist eine Kalibrierkurve aufgenommen worden (Figur 2), die zur Ausbeuteberechnung der in den Beispielen 1 bis 6 durchgeführten Versuche im kleineren Maßstab diente (vgl. Beispiel 1-6).

**Tabelle 7**: Katalysator: $nbPEt_2$ (1 mol-%, bezogen auf HDI) Reaktionstemperatur: 30°C, semi-kontinuierliche Reaktionsführung

| Bsp. 7- | R-Zeit [hh:mm] | $n_D^{20}$ | rel. Reaktivität | Ausbeute [%] | NCO-Gehalt | Viskosität [mPas] bei 23°C | Farbzahl [APHA] | fr. HDI [%] | U / T |
|---|---|---|---|---|---|---|---|---|---|
| 0 | 24:35 | 1,4671 | 100% | 39,6% | 21,5 | 105 | 27 | 0,10 | 5,3 |
| A | 20:20 | 1,4675 | 107% | 38,6% | 21,3 | 100 | 24 | 0,08 | 6,8 |
| B | 18:45 | 1,4694 | 98% | 46,0% | 20,9 | 130 | 33 | 0,09 | 5,1 |
| C | 69:43 | 1,4843 | 75% | 67,2% | 17,5 | 565 | 24 | 0,10 | 3,6 |
| D | 22:08 | 1,4652 | 78% | 35,2% | 21,8 | 102 | 51 | 0,11 | 5,9 |
| E | 22:43 | 1,4653 | 82% | 35,2% | 22,1 | 85 | 42 | 0,10 | 6,2 |
| F | 22:00 | 1,4640 | 79% | 32,4% | 21,9 | 76 | 40 | 0,10 | 6,8 |
| G | 22:36 | 1,4635 | 73% | 31,5% | 22,1 | 72 | 35 | 0,09 | 7,2 |
| H | 70:59 | 1,4771 | 55% | 58,9% | 18,9 | 218 | 13 | 0,11 | 4,3 |
| I | 22:44 | 1,4630 | 68% | 29,1% | 22,4 | 72 | 27 | 0,10 | 7,1 |

(fortgesetzt)

| Bsp. 7- | R-Zeit [hh:mm] | $n_D^{20}$ | rel. Reaktivität | Ausbeute [%] | NCO-Gehalt | Viskosität [mPas] bei 23°C | Farbzahl [APHA] | fr. HDI [%] | U / T |
|---|---|---|---|---|---|---|---|---|---|
| J | 22:42 | 1,4624 | 68% | 27,9% | 22,4 | 72 | 26 | 0,12 | 7,0 |
| K | 23:26 | 1,4627 | 65% | 27,4% | 22,5 | 68 | 17 | 0,08 | 7,1 |
| L | 28:17 | 1,4633 | 60% | 30,9% | 22,2 | 72 | 11 | 0,10 | 6,9 |
| M | 66:09 | 1,4724 | 50% | 49,2% | 19,5 | 140 | 9 | 0,16 | 5,0 |
| N | 22:50 | 1,4618 | 59% | 26,2% | 22,5 | 68 | 25 | 0,11 | 7,3 |
| O | 21:00 | 1,4618 | 64% | 25,7% | 23,0 | 62 | 24 | 0,25 | 7,4 |
| P | 20:27 | 1,4602 | 58% | 22,1 % | 23,1 | 64 | 30 | 0,07 | 8,0 |
| Q | 22:38 | 1,4608 | 55% | 22,7% | 23,1 | 58 | 19 | 0,08 | 7,7 |
| R | 70:31 | 1,4725 | 47% | 50,1% | 20,0 | 136 | 13 | 0,13 | 5,1 |

**Beispiel 8, Vergleichsbeispiel**

Katalysator: *cyclo*-Hex-P-*n*-$Hex_2$ (0,2 mol-%, bezogen auf HDI); Reaktionstemperatur: 40°C

Es wurde in Analogie zu Beispiel 7 verfahren, mit dem Unterschied, dass als Katalysator 3,6 g Cyclohexyl-di-n-hexylphosphin verwendet wurde. Die Versuchsdaten sind in Tabelle 8 aufgeführt.

Wie man sofort erkennt, ist die Uretdion-Selektivität des Cyclohexyl-di-n-hexylphosphins - bei vergleichbarem Umsatz - wesentlich geringer, als die des strukturell sehr ähnlichen, erfindungsgemäßen Norbornylderivates aus Beispiel 1.

Die deutlich geringere Standzeit des Cyclohexyl-di-n-hexylphosphins gegenüber Norbornyl-diethylphosphin geht sehr gut aus dem Vergleich der Figuren 1 und 3 hervor.

**Tabelle 8**: Katalysator: *cyclo*-Hex-P-*n*-$Hex_2$ (0,2 mol-%, bezogen auf HDI) Reaktionstemperatur: 40°C, semi-kontinuierliche Reaktionsführung

| Bsp. 8- | R-Zeit [hh: mm] | $n_D^{20}$ | rel. Reaktivität | Ausbeute [%] | NCO-Gehalt | Viskosität [mPas] bei 23°C | Farbzahl [APHA] | fr. HDI [%] | U/T |
|---|---|---|---|---|---|---|---|---|---|
| 0 | 18:41 | 1,4643 | 100,0% | 32,8% | 22,4 | 108 | 81 | 0,17 | 3,0 |
| A | 22:58 | 1,4656 | 83,3% | 35,0% | 22,1 | 130 | 40 | 0,16 | 3,1 |
| B | 22:15 | 1,4629 | 68,0% | 29,2% | 22,7 | 112 | 45 | 0,12 | 3,3 |
| C | 22:37 | 1,4609 | 55,3% | 23,9% | 22,8 | 97 | 35 | 0,08 | 3,3 |
| D | 05:26 | 1,4551 | 52,7% | 7,1% | 24,0 | 98 | 43 | 0,09 | 4,3 |
| E | 16:51 | 1,4583 | 42,0% | 14,9% | 23,2 | 100 | 30 | 0,11 | 3,3 |
| F | 23:40 | 1,4598 | 42,7% | 19,3% | 23,0 | 96 | 27 | 0,07 | 3,3 |
| G | 19:52 | 1,4587 | 40,0% | 17,2% | 23,6 | 94 | 17 | 0,08 | 2,0 |
| H | 21:13 | 1,4584 | 38,0% | 12,2% | 23,8 | 90 | 27 | 0,08 | 3,6 |
| I | 03:31 | 1,4553 | 45,3% | 6,8% | 23,9 | 71 | 42 | 0,12 | 6,4 |
| J | 25:35 | 1,4574 | 26,0% | 13,4% | 23,8 | 85 | 40 | 0,13 | 3,7 |
| K | 43:05 | 1,4590 | 18,0% | 16,8% | 22,9 | 96 | 63 | 0,08 | 3,6 |
| L | 56:00 | 1,4565 | 9,3% | 10,7% | 23,9 | 95 | 66 | 0,07 | 3,2 |

**Beispiel 9, erfindungsgemäß**

Katalysator: $nb_2PEt$ (2,5 mol-%, bezogen auf HDI); Reaktionstemperatur: 30°C

Es wurde in Analogie zu Beispiel 7 verfahren, mit dem Unterschied, dass als Katalysator $nb_2PEt$ verwendet wurde. Die Daten sind in Tabelle 9 aufgeführt.

**Tabelle 9**: Katalysator: $nb_2PEt$ (2,5 mol-%, bezogen auf HDI) Reaktionstemperatur: 30°C, semi-kontinuierliche Reaktionsführung

| Bsp. 9- | Reaktionszeit [hh:mm] | $n_D^{20}$ | rel. Reaktivität | Ausbeute [%] | NCO-Gehalt | Viskosität [mPas] bei 23°C | Farbzahl [APHA] | fr. HDI [%] | U/T |
|---|---|---|---|---|---|---|---|---|---|
| 0 | 24:13 | 1,4625 | 100,0% | 21,9% | 23,0 | 53 | 91 | 0,13 | 16,0 |
| A | 22:02 | 1,4627 | 97,0% | 19,5% | 23,1 | 56 | 55 | 0,08 | 18,4 |
| C | 20:55 | 1,4620 | 95,5% | 17,9% | 23,2 | 67 | 57 | 0,10 | 19,8 |
| D | 22:08 | 1,4622 | 91,0% | 17,9% | 23,1 | 60 | 43 | 0,07 | 18,9 |
| E | 22:05 | 1,4622 | 89,6% | 17,9% | 23,1 | 65 | 43 | 0,09 | 19,6 |
| F | 22:22 | 1,4620 | 85,1% | 17,8% | 23,1 | 61 | 59 | 0,08 | 19,2 |
| G | 22:02 | 1,4625 | 88,1% | 18,7% | 23,2 | 64 | 54 | 0,11 | n.b. |
| H | 22:07 | 1,4624 | 86,6% | 18,8% | 23,2 | 69 | 40 | 0,09 | n.b. |
| I | 70:18 | 1,4712 | 74,6% | 42,3% | 20,8 | 95 | 24 | 0,08 | 15,0 |
| J | 21:34 | 1,4618 | 85,1% | 17,4% | 23,1 | 62 | 79 | 0,08 | n.b. |
| K | 22:22 | 1,4615 | 79,1% | 15,7% | 23,2 | 66 | 50 | 0,07 | n.b. |
| L | 22:11 | 1,4617 | 79,1% | 17,1% | 23,3 | 68 | 60 | 0,31 | n.b. |
| M | 28:08 | 1,4630 | 77,6% | 21,0% | 23,0 | 67 | 27 | 0,07 | n.b. |
| N | 64:09 | 1,4692 | 70,1% | 38,3% | 21,1 | 81 | 21 | 0,06 | 16,9 |
| O | 24:30 | 1,4613 | 70,1% | 17,5% | 23,0 | 65 | 48 | 0,07 | n.b. |
| P | 24:20 | 1,4616 | 76,1% | 11,9% | 22,3 | 98 | 68 | 0,08 | n.b. |

n.b.: nicht bestimmt

**Patentansprüche**

1. Verwendung von Phosphinen gemäß Formel (1)

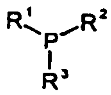

(I)

wobei

$R^1$ ein gegebenenfalls ein oder mehrfach $C_1$-$C_{12}$ Alkyl- oder Alkoxy-substituierter, bicyclischer, cycloaliphatischer $C_4$-$C_{20}$-Rest ist und
$R^2$, $R^3$ unabhängig voneinander ein gegebenenfalls ein oder mehrfach $C_1$-$C_{12}$ Alkyl- oder Alkoxysubstituierter mono- oder bicyclischer, cycloaliphatischer $C_4$-$C_{20}$-Rest oder ein linear oder vertweigt aliphatischer $C_1$-$C_{20}$-Rest ist.

bei der Dimerisierung von Isocyanaten.

2. Verfahren zur Dimerisierung von Isocyanaten, bei dem

a) mindestens ein organisches Isocyanat einer NCO-Funktionalität ≥ 2,
b) ein Katalysator enthaltend mindestens ein Phosphin gemäß Formel (1)

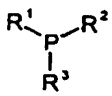

(I)

wobei

$R^1$ ein gegebenenfalls ein oder mehrfach $C_1$-$C_{12}$ Alkyl- oder Alkoxy-substituierter, bicyclischer, cycloaliphatischer $C_4$-$C_{20}$-Rest ist und
$R^2$, $R^3$ unabhängig voneinander ein gegebenenfalls ein oder mehrfach $C_1$-$C_{12}$ Alkyl- oder Alkoxysubstituierter mono- oder bicyclischer, cycloaliphatischer $C_4$-$C_{20}$-Rest oder ein linear oder vertweigt aliphatischer $C_1$-$C_{20}$-Rest ist.

c) optional Lösemittel und
d) optional Additive
zur Reaktion gebracht werden.

3. Verfahren zur Dimerisierung von Isocyanaten nach Anspruch 2, **dadurch gekennzeichnet, dass** die Dimerisierung bei einer Temperatur von 0 bis 150°C bis zu einem Umsatz der NCO-Gruppen von 5 bis 90 % geführt und danach abgebrochen wird.

4. Verfahren zur Dimerisierung von Isocyanaten nach Anspruch 2 oder 3, **dadurch gekennzeichnet, dass** in Komponente a) aliphatische, cycloaliphatische oder araliphatische Di-oder Polyisocyanaten einer NCO-Funktionalität ≥ 2 eingesetzt werden.

## Claims

1. Use of phosphines according to formula (I)

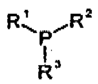

(I)

wherein

$R^1$ is a bicyclic, cycloaliphatic $C_4$-$C_{20}$ radical optionally substituted with one or more $C_1$-$C_{12}$ alkyl or alkoxy groups,
$R^2$, $R^3$ independently of each other are a monocyclic or bicyclic, cycloaliphatic $C_4$-$C_{20}$ radical, optionally substituted with one or more $C_1$-$C_{12}$ alkyl or alkoxy groups, or a linear or branched aliphatic $C_1$-$C_{20}$ radical.

2. Process for the dimerisation of isocyanates, wherein

a) at least one organic isocyanate having an NCO functionality of ≥ 2,
b) a catalyst containing at least one phosphine according to formula (I)

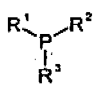

(I)

wherein

$R^1$ is a bicyclic, cycloaliphatic $C_4$-$C_{20}$ radical optionally substituted with one or more $C_1$-$C_{12}$ alkyl or alkoxy groups,
$R^2$, $R^3$ independently of each other are a monocyclic or bicyclic, cycloaliphatic $C_4$-$C_{20}$ radical, optionally substituted with one or more $C_1$-$C_{12}$ alkyl or alkoxy groups, or a linear or branched aliphatic $C_1$-$C_{20}$ radical,

c) optionally solvents and
d) optionally additives
are reacted together.

**3.** Process for the dimerisation of isocyanates according to claim 2, **characterised in that** the dimerisation is performed at a temperature of 0 to 150°C until an NCO group conversion rate of 5 to 90% is achieved and is then terminated.

**4.** Process for the dimerisation of isocyanates according to claim 2 or 3, **characterised in that** aliphatic, cycloaliphatic or araliphatic di- or polyisocyanates having an NCO functionality of ≥ 2 are used in component a).

## Revendications

**1.** Utilisation de phosphines selon la formule (I)

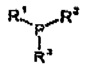 (I)

dans laquelle

$R^1$ représente un radical en $C_4$-$C_{20}$ bicyclique, cycloaliphatique, le cas échéant mono- ou polysubstitué par un alkyle en $C_1$-$C_{12}$ ou un alcoxy et
$R^2$ et $R^3$ désignent indépendamment l'un de l'autre un radical en $C_4$-$C_{20}$ mono- ou bicyclique, cyaloaliphatique, le cas échéant mono- ou polysubstitué par un alkyle en $C_1$-$C_{12}$ ou un alcoxy, ou un radical en $C_1$-$C_{20}$ aliphatique, linéaire ou ramifié,

pour la dimérisation d'isocyanates.

**2.** Procédé de dimérisation d'isocyanates, dans lequel sont mis en réaction

a) au moins un isocyanate organique possédant une fonctionnalité NCO ≥ 2,
b) un catalyseur contenant au moins une phosphine selon la formule (I)

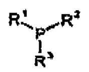 (I)

dans laquelle

$R^1$ représente un radical en $C_4$-$C_{20}$ bicyclique, cycloaliphatique, le cas échéant mono- ou polysubstitué par un alkyle en $C_1$-$C_{12}$ ou un alcoxy et
$R^2$ et $R^3$ désignent indépendamment l'un de l'autre un radical en $C_4$-$C_{20}$ mono- ou bicyclique, cycloaliphatique, le cas échéant mono- ou polysubstitué par un alkyle en $C_1$-$C_{12}$ ou un alcoxy, ou un radical en $C_{1-}C_{20}$ aliphatique, linéaire ou ramifié,

c) des solvants optionnels et
d) des additifs optionnels.

**3.** Procédé de dimérisation d'isocyanates selon la revendication 2, **caractérisé en ce que** la dimérisation est opérée à une température comprise entre 0 et 150 °C jusqu'à une conversion des groupes NCO comprise entre 5 et 90 %, puis interrompue.

**4.** Procédé de dimérisation d'isocyanates selon la revendication 2 ou 3, **caractérisé en ce que** sont mis en oeuvre dans le composant a), des di- ou des polyisocyanates aliphatiques, cycloaliphatiques ou araliphatiques possédant une fonctionnalité NCO ≥ 2.

*Fig. 1*

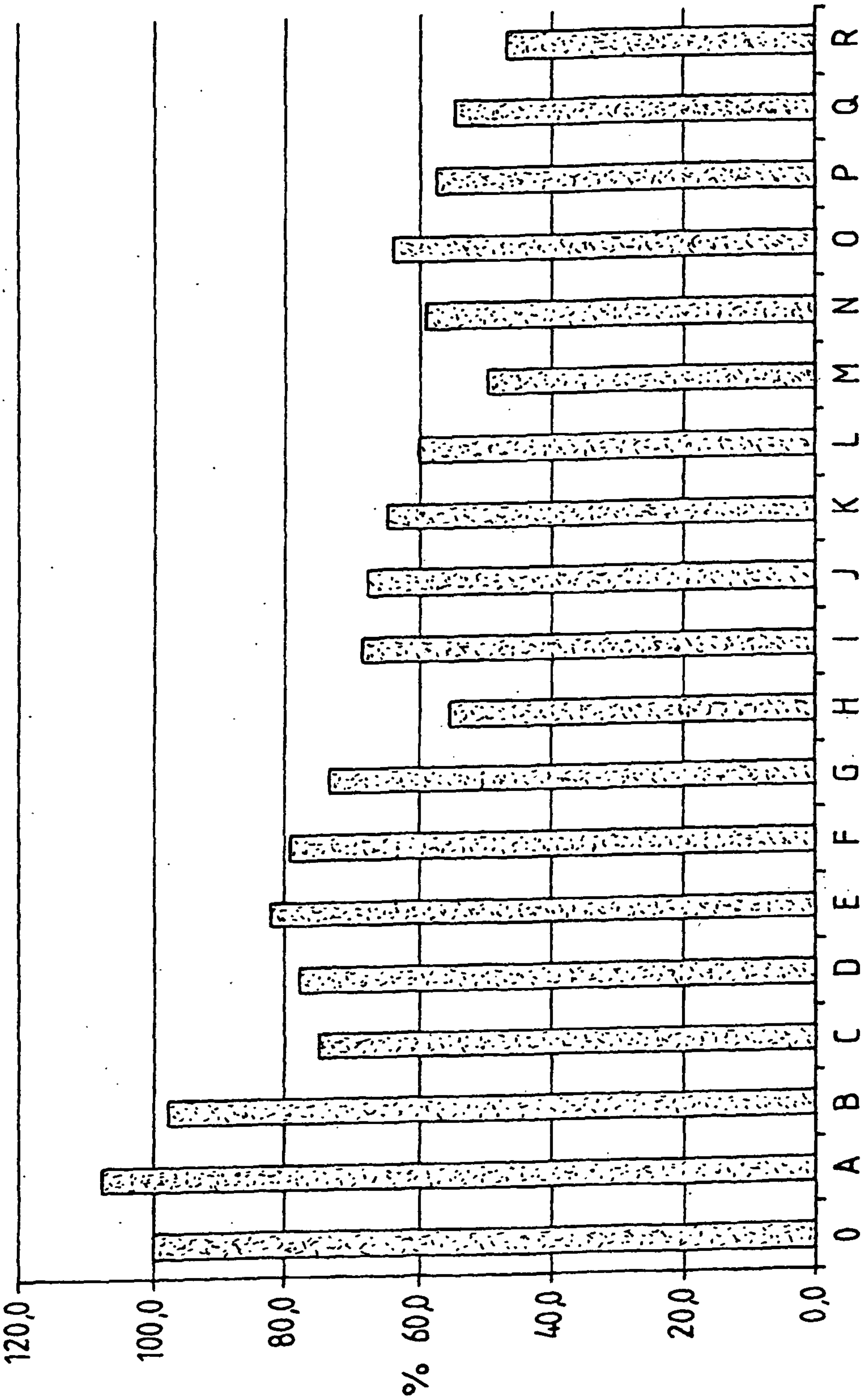

**Fig. 2**

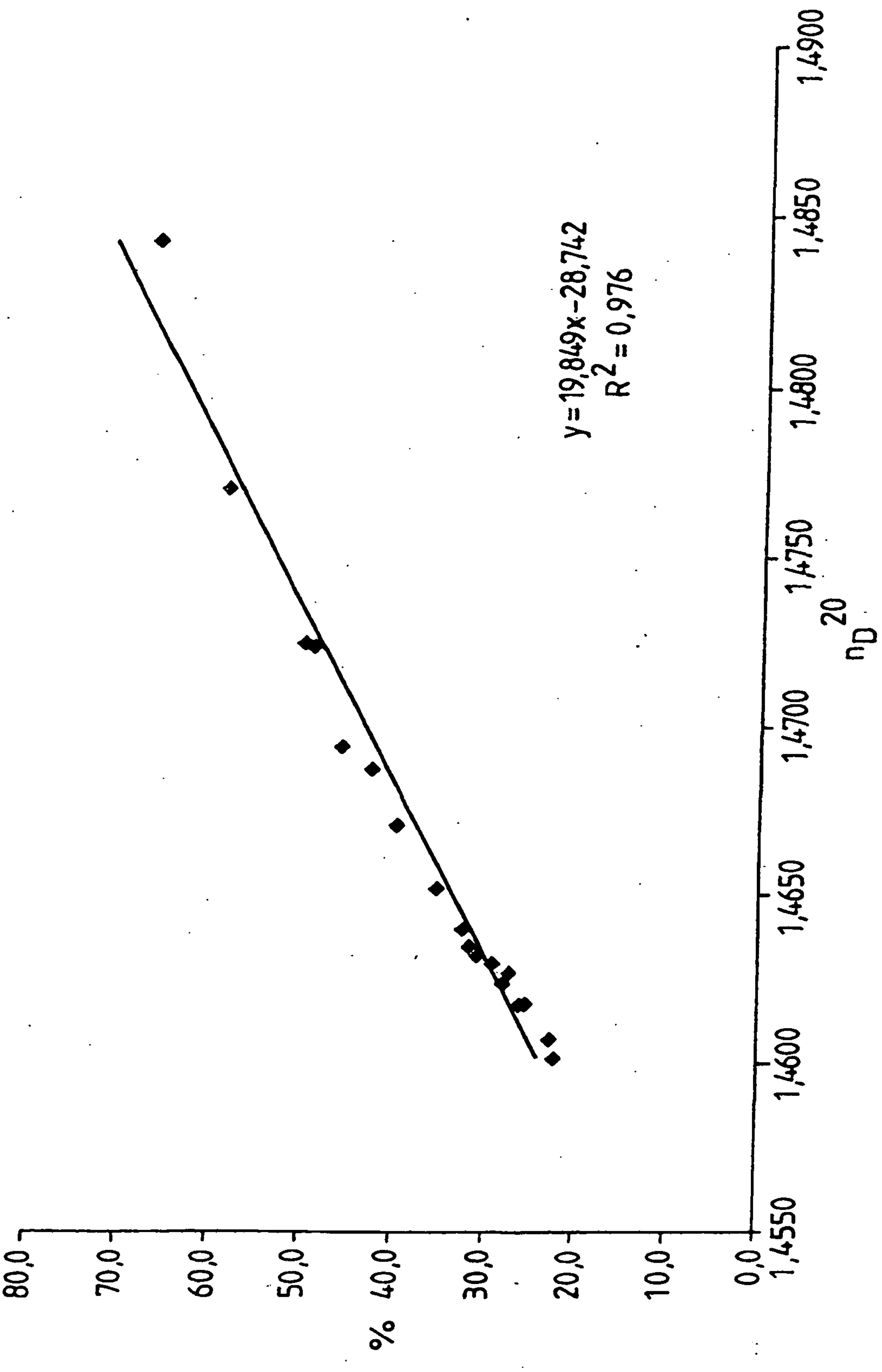
%
80,0
70,0
60,0
50,0
40,0
30,0
20,0
10,0
0,0
1,4550
1,4600
1,4650
1,4700
1,4750
1,4800
1,4850
1,4900
$n_D^{20}$
y = 19,849x - 28,742
$R^2$ = 0,976

Fig. 3

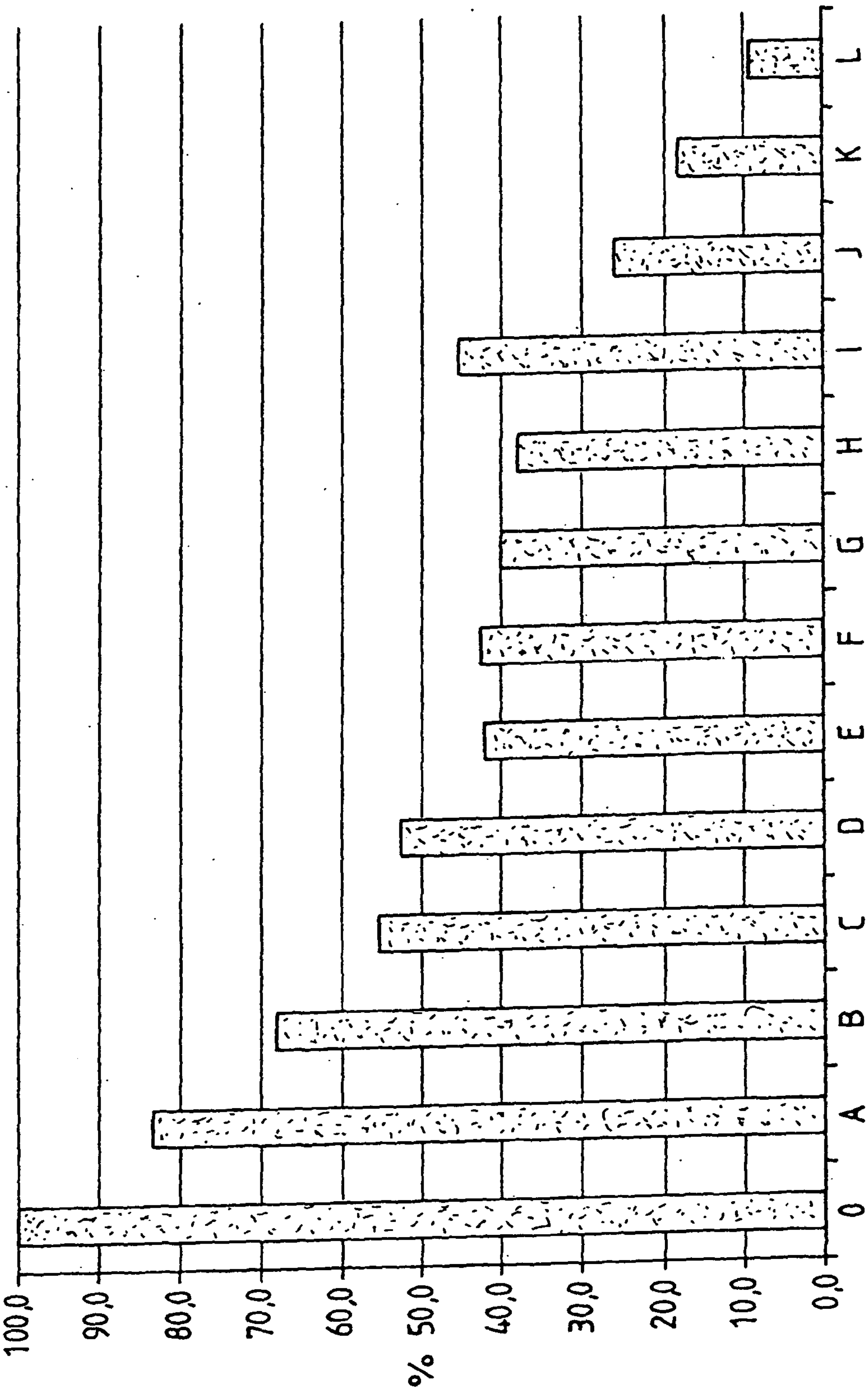

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 3030513 A
- DE 3437635 A
- DE 3739549 A
- DE 1670720 A
- DE 10254878 A
- DE 1670667 A
- DE 1934763 A
- DE 1954093 A
- US 4614785 A


### In der Beschreibung aufgeführte Nicht-Patentliteratur


- *J. Prakt. Chem./Chem. Ztg.,* 1994, vol. 336, 185-200
- *J. Org. Chem.,* 1961, vol. 26 (513), 8-5145